# EUROPEAN PATENT APPLICATION

(11) **EP 1 297 848 A1**
(43) Date of publication of application: **02.04.2003**
(21) Application number: 01938673.9
(22) Date of filing: 14.06.2001
(51) Int. Cl.: A61K 45/00, A61K 38/38, A61K 39/395, A61K 31/711, A61K 31/795, A61K 31/787, A61K 31/721, A61P 15/00

(54) **PREVENTIVES/REMEDIES FOR GRANULOMA**

(30) Priority: 16.06.2000 JP 2000185942
(71) Applicant: CHUGAI SEIYAKU KABUSHIKI KAISHA, Tokyo, 115-8543 (JP)
(72) Inventor: SUZUKI, Hiroshi, c/o CHUGAI SEIYAKU K K, Gotemba-shi, Shizuoka 412- 8513 (JP); JISHAGE, Kou-ichi, c/o CHUGAI SEIYAKU K K, Gotemba-shi, Shizuoka 41 2-8513 (JP)
(74) Representative: Maschio, Antonio
(86) International application number: JP0105082
(87) International publication number: WO01095938

(57) **Abstract**

A preventive or therapeutic agent for granuloma, specifically for granuloma occurring after ligation of a deferent duct, comprising an antagonist against the scavenger receptor of macrophage, such as antibody, as an active ingredient.

## Description

### Field of Invention

The present invention relates to a preventive and/or therapeutic agent for granuloma, specifically granuloma occurring after ligation of a deferent duct.

### Background Art

It is believed that, after ligation of deferent duct, one of the sterilization surgeries, sperm are processed through decomposition and dissolution in the epididymal cavity and phagocytosis by macrophages. Phagocytosis by macrophages is generally known to occur through scavenger receptors expressed on the cell surface of macrophages, and the like.

On the other hand, as a side effect after ligation of a deferent duct, sperm granuloma is known to occur in a small percentage of the patients and to be frequently accompanied by pain. Though anti-inflammatory agents have been used for the treatment, little is known on the cause and the mechanism of the onset of the symptoms, and hence there are no known fully effective preventive or therapeutic agents.

### Disclosure of the Invention

Thus, the present invention intends to provide a novel preventive or therapeutic agent for granuloma, specifically for sperm granuloma occurring after ligation of a deferent duct.

In an effort to solve the above problems, the present inventors have found that, using scavenger receptor knockout mice in which the expression of scavenger receptors has been blocked by gene engineering and normal mice, granuloma does not occur in the knockout mice after ligation of deferent duct was performed on both mice, and thereby have completed the present invention. Thus, no occurrence of sperm granuloma after ligation of deferent duct in the scavenger receptor knockout mice allows one to estimate reasonably that sperm granuloma occurring after ligation of deferent duct may be prevented or treated by an antagonist against scavenger receptors.

Thus, the present invention intends to provide a preventive and/or therapeutic agent for granuloma comprising an antagonist against the scavenger receptor of macrophages as an active ingredient, and more specifically a preventive and/or therapeutic agent for sperm granuloma occurring after ligation of deferent duct, said agent comprising as an active ingredient an antagonist against the scavenger receptor of macrophages.

Antagonists against scavenger receptors for use in the present invention include antibody against the scavenger receptor such as monoclonal antibody, chimeric antibody, humanized antibody and single chain antibody, as well as a fragment of the above antibodies capable of biding to scavenger receptors.

Furthermore, as the above antagonists, there can be mentioned antisense nucleic acids against nucleic acids encoding scavenger receptors, low molecular weight compounds, and the like.

### Brief Explanation of the Drawings

Fig. 1 is a graph showing changes with time in the sperm count of the wild type mice and the scavenger receptor knockout mice after ligation of a deferent duct.
Fig. 2 is a graph showing changes with time in the weight of testis of the wild type mice and the scavenger receptor knockout mice after ligation of a deferent duct. Embodiment for Carrying Out the Invention

As described below in the Example, the present inventors have found that when ligation of deferent duct was performed on scavenger receptor mice in which the expression of scavenger receptor A (SR-A) has been deleted by genetic engineering and on normal mice, and then the occurrence of granuloma on them was examined, granuloma occurred in the normal mice while no granuloma occurred in the scavenger receptor knockout mice though there was no substantial difference in the rate of sperm formation and the weight of testis, and thereby have found that the occurrence of sperm granuloma occurring after ligation of deferent duct can be prevented by blocking the scavenger receptor.

As antagonists against scavenger receptors, there can be mentioned antibodies against scavenger receptors. Macrophage scavenger receptor (MSR-A) is a trimer membrane type glycoprotein with a molecular weight of about 22 kD, and is comprised of a C-terminal end-specific domain, a collagen-like domain, an α-helical coiled coil domain and a spacer domain on the outside of the cell; the transmembrane domain; and the cytoplasmic domain (Kodama T. et al., Nature 343:531-535 (1990; Matsumoto A. et al., Proc. Natl. Acad. Sci. USA 87:9133-9137 (1990)), and is expressed on the cell surface of macrophage cell lines.

Thus, in the preparation of antibodies, macrophages having scavenger receptors expressed on the cell surface and the cell membrane fractions thereof, the isolated scavenger receptors, specifically the extracellular domains thereof, recombinant scavenger receptor protein produced by genetic engineering, specifically the extracellular domains thereof, for example C-terminal end-specific domain and the like can be used as the immunogen.

Polyclonal antibodies against scavenger receptors can be obtained from the serum of a non-human animal such as a rabbit and a goat immunized with the above immunogens. Monoclonal antibodies can also be produced by methods well known in the art. For example, experimental animals such as mice are immunized with the above immunogen, antibody-producing cells such as spleen cells are harvested, which are then fused with a myeloma cell line such as SP/2 to obtain a hybridoma producing the monoclonal antibody of interest.

Humanized antibodies are prepared by replacing the complementarity determining region (CDR) in the variable region (V region) of human antibody heavy chain (H chain) and light chain (L chain) with the corresponding CDR of monoclonal antibody of a non-human animal such as mice and, as the framework region (FR) of the constant region (C region) and the V region is derived from the human antibody and the CDR is only derived from monoclonal antibody of the non-human animal, it has an advantage of having a very low immunogenicity to humans.

The method of preparing humanized antibodies is well known and, for example, cDNA encoding the V region of the H chain and the L chain of a mouse monoclonal antibody to a scavenger receptor is cloned to determine the amino acid sequence, and CDR and FR are differentiated, and the variable region of human monoclonal antibody having a high homology with the amino acid sequence of mouse FR region is selected, and the CDR coding region of cDNA encoding the V region is substituted for the CDR coding region of mouse monoclonal antibody using a known gene engineering technology, and a vector containing the H chain coding region thus prepared and a vector containing the L chain coding region are expressed in a host animal cell and the formed H chain protein and the L chain protein are assembled.

Chimeric antibodies are prepared by ligating the C region of a human monoclonal antibody and the V region of an animal monoclonal antibody for each of the H chain and the L chain, and in the present invention, the V region of a mouse monoclonal antibody to a scavenger receptor is ligated to the C region of an optional human antibody. The method of preparing chimeric antibodies is also well known in the art and, for example, from DNA encoding a mouse monoclonal antibody to a scavenger receptor, a DNA fragment encoding the V region is cloned, which is then ligated to a DNA fragment encoding the C region of a human antibody, an expression vector is prepared for each of the H chain and the L chain, which are then expressed in a host cell, and the formed H chain protein and the chimeric L chain protein are assembled.

Single chain antibodies are prepared by ligating the H chain V region of a monoclonal antibody of mice etc., a humanized monoclonal antibody, or a chimeric antibody to a scavenger receptor to the L chain V region via a suitable spacer. The method of preparing single chain antibodies is also well known, and DNA encoding the V region of the H chain and the L chain of the above monoclonal antibody, chimeric antibody or a humanized antibody is obtained, which is ligated via DNA encoding a linker, and an expression vector containing this is introduced into an animal host cell in which it is expressed.

An alternative embodiment of an antagonist to a macrophage scavenger receptor may be an antisense nucleic acid to a nucleic acid encoding the scavenger receptor. The gene encoding the human macrophage scavenger receptor has been cloned (Matusmoto, A. et al., Proc. Natl. Acad. Sci. USA 87:9133-9137 (1990)), and a nucleic acid complementary to some of the gene sequence may be used as an antisense.

The antagonist of the present invention may be a low molecular weight compound.

As examples of scavenger receptor antagonists, there are known polyvinyl sulfate, polyinosinic acid, polyxanthinylic acid, polyguanylic acid, poly G, I(1:1) (poly G, I(1:1)), dextran sulfate, fucoidin, carragheenan, bovine sulfatides, maleylated LDL, maleylated albumin, and the like.

Examples of antagonists to macrophage scavenger receptors for use in the present invention have been described in PCT publication WO 200006147, WO 2000003704, WO 9907382, Japanese Unexamined Patent Publication (Kokai) No. 11-246512, Japanese Unexamined Patent Publication (Kokai) No. 11-246511, J. Pharmacol. Exp. Ther. (1999), 289 (3):1277-1285, M.S. Brown, S.K. Basu, J.R. Falck, Y.K. Ho, and J.L. Goldstein, The Scavenger Cell Pathway for Lipoprotein Degradation: Specificity of the Binding Site That Mediates the Uptake of Negatively-Charged LDL by Macrophages, Journal of Supramolecular Structure, 13:67-81 (1980), and the like.

The preventive and/or therapeutic agent of the present invention can be administered either orally or parenterally, and preferably the antibody is administered by injection, for example intravenous injection.

The dosage of the antibody as the antagonist to the scavenger receptor may be selected as appropriate depending on the weight and the symptom of the patient, and it is, for example, 0.1-1000 mg per kg of body weight per day, and preferably 1-300 mg/kg.

### Examples

The present invention will now be explained in more detail with reference to following Example.

### Example

### Method

Male scavenger receptor A (SR-A) knockout mice (Suzuki H. et al., Nature 386:292-296 (1997)) and wild type mice were anesthetized with the injection of sodium pentobarbital (Nembutal, Abbot Laboratories, U.S.A.) and were subjected to surgery. A small vertical abdominal midline incision was made. Each seminal duct was ligated twice using 5-zero silk (Matsuda Sutures, Tokyo).

One to four weeks after the ligature of the seminal duct, the animals were sacrificed, and the testis and the epididymis accompanied by the seminal duct were extracted. The weight of the testis was found, and sperm count per day was determined by the method of Joyce et al., J. Androl. 14:448-455 (1993). The epididymis accompanied by the seminal duct was fixed in a neutrally buffered 20% formalin solution or a 2% periodate-lysine-paraformaldehyde for hematoxylin-eosin stain and immunohistochemistry, respectively.

For immunohistochemistry, the following antibodies were used. 2F8: a mouse monoclonal antibody (Fraser et al., Nature 364:343-346 (1993)) to mouse SR-A, F4/80: a mouse monoclonal antibody (Leener PJ. et al., J. Immunol. Methods, 175:5-19 (1992)) to macrophage, and FA/11: a monoclonal antibody (CD68) (Holness C.D. et al., J. Biol. Chem. 30:9661-9666 (1993)) to rodent macrosialin

All animals were kept in a chamber that was light-controlled (lighting during 0500-1900h) and air-controlled. The temperature of the animal chamber was set at 24 ± 2°C, and the humidity was controlled at 55 ± 10%. The animals used in this experiment were handled according to the Guiding Principles for the Care and Use of Research Animals (Chugai Pharmaceutical Co., Ltd.). Result

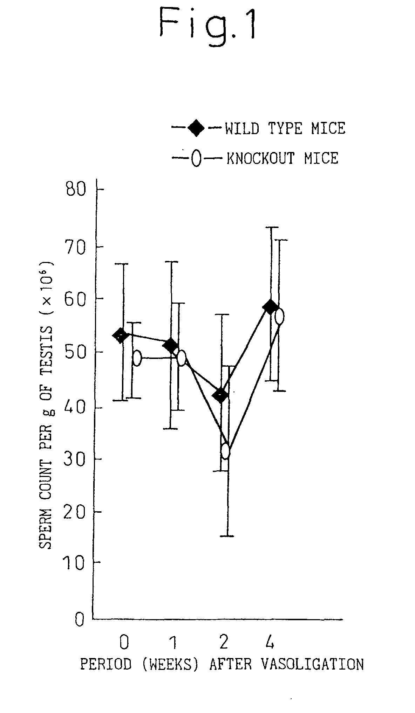

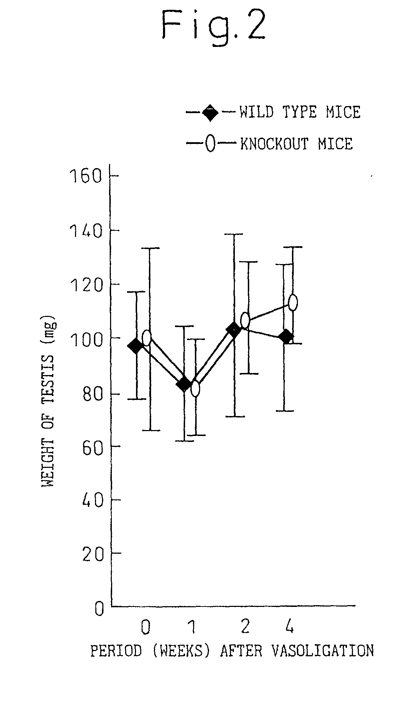

As shown in Fig. 1 and Fig. 2, there was no substantial difference in the weight of testis and the sperm count per day during the observation period between the knockout mice and the wild type mice. In the knockout mice the sperm count per day significantly decreased two weeks after the surgery, and four weeks after the surgery it restored to the value of the control mice that did not receive surgery. The value of the sperm count per day in the knockout mice two weeks after the surgery was significantly different from the value of the sperm count per day of the wild type mice two weeks after the surgery.

In the wild type mice, during the observation period, about half of the mice that received surgery suffered from granuloma. The granuloma was located mostly in the corpus epididymis and the cauda epididymis of the wild type mice. However, none of the 17 SR-A knockout mice that received surgery had sperm granuloma (Table 1). If one of the causes of granuloma is the extravasation of sperms from the duct of epididymis as a result of increased pressure in the seminal duct following its blocking, the SR-A knockout mice that were vasoligated should exhibit sperm granuloma as can be observed in the wild type mice that of which deferent duct was ligated.

While the sperm count per day and the weight of testis in the SR-A knockout mice after ligation of deferent duct were not different from those of the wild type mice (Fig. 1 and Fig. 2), the SR-A knockout mice, whose deferent ducts had been ligated, did not form granuloma during the observation period. Thus, it is unlikely that the increased pressure in the seminal duct caused the overflow of sperm leading to the formation of sperm granuloma.

Table 2 shows the expression of scavenger receptor antigen in the head, the body and the tail portions of epididymis of the wild type mice and the granuloma of the epididymis. The basal cells in the head, the body and the tail portions of epididymis were positive for F4/80 but were negative for 2F8. The SR-A antigen was expressed on macrophages in the interstitial tissue in all portions of the epididymis.

The expression of the SR-A antigen was not observed or was weakly observed in the basal cells and the epithelial cells of the head, the body and the tail portions of epididymis. Phagocytic cells responsible for the disappearance of sperms were not present in the interstitial tissue or the lumen of the epididymis, but granuloma proved strongly positive for 2F8. These observations suggest that the antigenicity of epithelial cells varies with the onset and development of granuloma (Table 2).

The above results indicate that scavenger receptor A is associated with the development of granuloma after ligation of deferent duct. Thus, the inhibition of scavenger receptor A activity is effective for prevention of formation and therapy of granuloma.

### Table 1

The formation of sperm granuloma in the wild type mice and the scavenger receptor knockout mice after ligation of deferent duct
The number of mice in which granuloma was formed / total number of knockout mice (%)

| Mice | Days after ligation of deferent duct | | | |
|---|---|---|---|---|
| | 7 days | 10 days | 14 days | one month |
| Knockout mice | 0/6(0) | 0/2(0) | 0/4(0) | 0/5(0) |
| Wild type mice | 2/5(40) | 3/4(75) | 2/3(7) | 2/6(33) |

### Table 2

The result of immunohistochemical detection of the expression of scavenger receptor in the epididymis of mice after ligation of deferent duct

| | | Antibody | | |
|---|---|---|---|---|
| | | 2F8 | F4/80 | FA11 |
| Head portion of epididymis | In the lumen | - | - | - |
| | Basal cells | - | + | - |
| | Epithelial cells | ± | ± | + |
| | Interstitial cells | + | + | + |
| Body portion of epididymis | In the lumen | - | - | - |
| | Basal cells | - | + | - |
| | Epithelial cells | - | - | - |
| | Interstitial cells | + | + | + |
| Tail portion of epididymis | In the lumen | - | - | - |
| | Basal cells | - | + | - |
| | Epithelial cells | - | - | - |
| | Interstitial cells | + | + | + |
| | Granuloma | + | + | + |

## Claims

1. A preventive and/or therapeutic agent for granuloma comprising an antagonist against the scavenger receptor of macrophage as an active ingredient.

2. A preventive and/or therapeutic agent according to claim 1 wherein said granuloma is a granuloma occurring after ligation of deferent duct.

3. A preventive and/or therapeutic agent according to claim 1 or 2 wherein said antagonist is an antibody against the scavenger receptor of macrophage.

4. A preventive and/or therapeutic agent according to claim 3 wherein said antibody is a monoclonal antibody, a chimeric antibody, a humanized antibody or single chain antibody, or a fragment of the above antibodies capable of biding to the scavenger receptor of macrophage.

5. A preventive and/or therapeutic agent according to claim 1 or 2 wherein said antagonist is an antisense nucleic acid against said scavenger receptor.

6. A preventive and/or therapeutic agent according to claim 1 or 2 wherein said antagonist is a low molecular weight compound.

7. A preventive and/or therapeutic agent according to claim 6 wherein said low molecular weight compound is selected from the group consisting of polyvinyl sulfate, polyinosinic acid, polyxanthinylic acid, polyguanylic acid, poly G, I(1:1) (poly G, I(1:1)), dextran sulfate, fucoidin, carragheenan, bovine sulfatides, maleylated LDL, and maleylated albumin.

8. The use as an active ingredient of an antagonist against the scavenger receptor of macrophage for the production of a preventive and/or therapeutic agent for granuloma.

9. The use according to claim 8 wherein said granuloma is granuloma occurring after ligation of deferent duct.

10. The use according to claim 8 or 9 wherein said antagonist is antibody to the scavenger receptor of macrophage.

11. The use according to claim 10 wherein said antibody is a monoclonal antibody, chimeric antibody, humanized antibody or single chain antibody, or a fragment of the above antibodies capable of binding to the scavenger receptor of macrophage.

12. The use according to claim 8 or 9 wherein said antagonist is an antisense nucleic acid against said scavenger receptor.

13. The use according to according to claim 8 or 9 wherein said antagonist is a low molecular weight compound.

14. The use according to according to claim 13 wherein said low molecular weight compound is selected from the group consisting of polyvinyl sulfate, polyinosinic acid, polyxanthinylic acid, polyguanylic acid, poly G, I(1:1) (poly G, I(1:1)), dextran sulfate, fucoidin, carragheenan, bovine sulfatides, maleylated LDL, and maleylated albumin.

15. A method of preventing and/or treating granuloma comprising an antagonist against the scavenger receptor of macrophage as an active ingredient.

16. A preventive and/or therapeutic method according to claim 15 wherein said granuloma is granuloma occurring after ligation of deferent duct.

17. A preventive and/or therapeutic method according to claim 15 or 16 wherein said antagonist is an antibody against the scavenger receptor of macrophage.

18. A preventive and/or therapeutic method according to claim 17 wherein said antibody is a monoclonal antibody, a chimeric antibody, a humanized antibody or a single chain antibody, or a fragment of the above antibodies capable of biding to the scavenger receptor of macrophage.

19. A preventive and/or therapeutic method according to claim 15 or 16 wherein said antagonist is an antisense nucleic acid against said scavenger receptor.

20. A preventive and/or therapeutic method according to claim 15 or 16 wherein said antagonist is a low molecular weight compound.

21. A preventive and/or therapeutic method according to claim 20 wherein said low molecular weight compound is selected from the group consisting of polyvinyl sulfate, polyinosinic acid, polyxanthinylic acid, polyguanylic acid, poly G, I(1:1) (poly G, I(1:1)), dextran sulfate, fucoidin, carragheenan, bovine sulfatides, maleylated LDL, and maleylated albumin.
